(19)

Europäisches
Patentamt
European
Patent Office
Office européen
des brevets

(11) **EP 4 542 561 A2**

(12) **EUROPEAN PATENT APPLICATION**

(43) Date of publication:
**23.04.2025 Bulletin 2025/17**

(21) Application number: **25162969.7**

(22) Date of filing: **20.04.2022**

(51) International Patent Classification (IPC):
**G16H 20/17** (2018.01)

(52) Cooperative Patent Classification (CPC):
**G16H 20/10; G16H 20/17**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.04.2021   US 202163177009 P**

(62) Document number(s) of the earlier application(s) in
accordance with Art. 76 EPC:
**22169007.6 / 4 080 516**

(71) Applicant: **Insulet Corporation
Acton, MA 01720 (US)**

(72) Inventors:
• **LEE, Joon Bok
Acton (US)**
• **ZHENG, Yibin
Hartland (US)**
• **O'CONNOR, Jason
Acton (US)**

(74) Representative: **Peterreins Schley
Patent- und Rechtsanwälte PartG mbB
Hermann-Sack-Straße 3
80331 München (DE)**

Remarks:
This application was filed on 11-03-2025 as a
divisional application to the application mentioned
under INID code 62.

(54) **IMPROVED AUTOMATIC DRUG DELIVERY SYSTEM FOR INDIVIDUALS WITH TYPE-2 DIABETES**

(57) Disclosed are a system, a device, processes, techniques and computer-readable media that set an insulin adjustment factor that is applied to a dose of a therapeutic exogenous substance. The adjustment factor accounts for production of endogenous insulin related to the therapeutic exogenous substance. A processor of an automatic drug delivery device may determine and administer the doses of the therapeutic exogenous substance according to a process in which an amount of a therapeutic exogenous substance to deliver is estimated.

An insulin adjustment factor utilizing an estimated relative therapeutic exogenous substance concentration may be determined. The processor may calculate a compensating dose of the therapeutic exogenous substance based on the estimated amount of the therapeutic exogenous substance to deliver that is modified by the insulin adjustment factor. The compensating dose of the therapeutic exogenous substance may be expelled from a reservoir by a pump mechanism.

FIG. 1

**EP 4 542 561 A2**

## Description

BACKGROUND

The etiology of Type-2 diabetes Mellitus (T2DM) is typically characterized by impaired insulin action (i.e., the endogenously produced insulin is inefficiently utilized by the type-2 diabetic's body), whereas Type-1 diabetes Mellitus (T1DM) is typically characterized by impaired, or essentially no, insulin production (by the type-1 diabetic's pancreas). Therefore, although people with T2DM may require external insulin delivery to maintain glucose control, they may still have endogenous insulin production capabilities which are regulated (primarily reduced) by the increases in the user's insulin concentrations. For instance, whereas the insulin action for people with T1DM may be considered to scale in a relatively linear manner with increasing insulin delivery, the insulin action of increased insulin delivery for T2DM patients typically fails to account for the reduction in endogenous insulin production and reduced delivery of the endogenous insulin.

Therefore, it would be beneficial if there were a system and processes that accounted for the reduced production of endogenous insulin.

BRIEF SUMMARY

This summary is provided to introduce a selection of concepts in a simplified form that are further described below in the Detailed Description. This summary is not intended to identify key features or essential features of the claimed subject matter, nor is it intended as an aid in determining the scope of the claimed subject matter.

In one aspect, an automatic drug delivery device, includes a processor, and a reservoir configured to contain a therapeutic exogenous medicament or substance. The automatic drug delivery device also includes a pump mechanism communicatively coupled to the processor and fluidically coupled to the reservoir. The automatic drug delivery device also includes a memory coupled to the processor and configured to store a substance delivery history and programming code that, when executed by the processor, configure the drug delivery device to estimate an amount of a therapeutic exogenous substance to deliver, determine an insulin adjustment factor utilizing an estimated therapeutic exogenous substance concentration, calculate a compensating dose of the therapeutic exogenous substance based on the estimated amount of the therapeutic exogenous substance to deliver that is adjusted by the insulin adjustment factor, and causing the calculated compensating dose of the therapeutic exogenous substance to be expelled from the reservoir by the pump mechanism.

In another aspect, a method includes estimating, by a processor of an automatic drug delivery device, an amount of a therapeutic exogenous substance to deliver, determining an insulin adjustment factor utilizing an estimated therapeutic exogenous substance concentration, calculating a compensating dose of the therapeutic exogenous substance based on the estimated amount of the therapeutic exogenous substance to deliver adjusted by an insulin adjustment factor, and causing the calculated compensating dose of the therapeutic exogenous substance to be expelled from a reservoir by a pump mechanism.

In a further aspect, an automatic drug delivery device includes processor, a reservoir configured to contain a therapeutic exogenous substance. The automatic drug delivery device also includes a pump mechanism communicatively coupled to the processor and fluidically coupled to the reservoir. The automatic drug delivery device also includes a memory coupled to the processor and configured to store a substance delivery history and programming code that, when executed by the processor, configure the drug delivery device to calculate a substance correction constraint based on an analyte measurement value, a user's target analyte measurement setting, and a substance sensitivity factor, determine a maximum substance concentration based on the substance correction constraint, determine an estimated therapeutic exogenous substance concentration based on delivery of the therapeutic exogenous substance over a set number of delivery cycles, determine an insulin adjustment factor using the maximum substance concentration and the estimated therapeutic exogenous substance concentration, determine an amount of a therapeutic exogenous substance to deliver based on an analyte measurement value of a user, calculate a compensating dose of the therapeutic exogenous substance based the amount of the therapeutic exogenous substance to deliver and the insulin adjustment factor, and causing the calculated compensating dose of the therapeutic exogenous substance to be expelled from the reservoir by the pump mechanism.

In yet another aspect, a method includes obtaining, by a processor of an automatic drug delivery device, an analyte measurement value of a user, calculating a substance correction constraint based on the analyte measurement value, a user's target analyte measurement setting, and a substance sensitivity factor, determining a maximum substance concentration based on the substance correction constraint, determining an insulin adjustment factor to account for production of an endogenous insulin related to a therapeutic exogenous substance administered by the automatic drug delivery device, calculating a compensating dose of the therapeutic exogenous substance based on the estimated amount of the therapeutic exogenous substance to deliver adjusted by an insulin adjustment factor, and expelling the compensating dose of the therapeutic exogenous substance from the reservoir by the pump mechanism.

In an additional aspect, a non-transitory computer-readable storage medium including instructions that, when executed by a processor, cause the processor to obtain, by a processor of an automatic drug delivery device, an analyte measurement value of a user, calculate a substance correction constraint based on the analyte measurement value, a user's target analyte measurement setting, and a substance sensitivity factor, determine a maximum substance concentration based on the substance correction constraint, determine a insulin adjustment factor utilizing the substance correction constraint, calculate a compensating dose of the therapeutic exogenous substance based on an estimated amount of the therapeutic exogenous substance to deliver adjusted by the insulin adjustment factor, and expel the compensating dose of the therapeutic exogenous substance from the reservoir by the pump mechanism.

In one more aspect, an automatic drug delivery system includes a processor, and a reservoir configured to contain a therapeutic exogenous substance. The automatic drug delivery system also includes a pump mechanism communicatively coupled to the processor and fluidically coupled to the reservoir. The automatic drug delivery system also includes a memory coupled to the processor and configured to store a substance delivery history and programming code that, when executed by the processor, configure the drug delivery device to estimate an amount of a therapeutic exogenous substance to deliver, determine an insulin adjustment factor utilizing an estimated therapeutic exogenous substance concentration, calculate a compensating dose of the therapeutic exogenous substance based on the estimated amount of the therapeutic exogenous substance to deliver that is adjusted by the insulin adjustment factor, and causing the calculated compensating dose of the therapeutic exogenous substance to be expelled from the reservoir by the pump mechanism.

BRIEF DESCRIPTION OF THE DRAWINGS

In the drawings, like reference characters generally refer to the same parts throughout the different views. In the following description, various embodiments of the present disclosure are described with reference to the following drawings, in which:

FIG. 1 illustrates a graphic illustrating an effect of exogenous insulin delivery on endogenous insulin production;
FIG. 2 illustrates a flowchart of an example process according to aspect of the disclosed subject matter;
FIG. 3 illustrates a flowchart of another example process according to aspect of the disclosed subject matter;
FIG. 4 illustrates a flowchart of an example of a detailed process according to aspect of the disclosed subject matter; and
FIG. 5 illustrates a functional block diagram of a system example suitable for implementing the example processes and techniques described herein.

DETAILED DESCRIPTION

Systems, devices, computer-readable medium and methods in accordance with the present disclosure will now be described more fully hereinafter with reference to the accompanying drawings, where one or more embodiments are shown. The systems, devices, and methods may be embodied in many different forms and are not to be construed as being limited to the embodiments set forth herein. Instead, these embodiments are provided so the disclosure will be thorough and complete, and will fully convey the scope of methods and devices to those skilled in the art. Each of the systems, devices, and methods disclosed herein provides one or more advantages over conventional systems, components, and methods.

Various examples provide a method, a system, a device and a computer-readable medium for responding to inputs provided by sensors, such as an analyte sensor, and users of an automatic drug delivery system. "Analyte sensor" may refer to a wearable device that includes sensing and measurement devices configured to detect different analytes in a user's body and particularly in the user's blood, as well as other analytes as described herein. The various devices and sensors that may be used to implement some specific examples may also be used to implement different therapeutic regimens using different drugs than those described in the specific examples.

In one example, the disclosed methods, system, devices or computer-readable medium may perform actions related to managing a user's blood glucose by incorporating an estimated therapeutic drug (such as insulin, glucagon, glucagon-like peptide, or the like) concentration term in automatic therapeutic delivery algorithms to account for endogenous production of an endogenous counterpart or complimentary substance, such as endogenous insulin, to the therapeutic drug, such as exogenous insulin. Said differently, when exogenous insulin is administered to the body, the body responds by not producing as much natural, or endogenous insulin. The effect of the exogenous insulin on the endogenous insulin and the overall insulin generated is an aspect of the present disclosure. A benefit of the disclosed devices, systems, techniques and processes implemented via medication delivery algorithms or applications (MDA), and specifically, AID algorithms for T2DM patients, accounted for the described changes in endogenous insulin delivery for optimal glucose control outcomes. In addition to providing for optimal glucose control outcomes, the following examples mitigate the inefficient delivery of exogenous insulin thereby reducing the waste of a valuable resource. Moreover, the described examples also potentially mitigate adverse physiological reactions and minimize continuous drastic physiological responses to underdelivering or overdelivering exogenous insulin.

The disclosed examples provide techniques that may be used with any additional algorithms or computer applications that manage blood glucose levels and insulin therapy. These algorithms and computer applications may be collectively referred to as "medication delivery algorithms" or "medication delivery applications" and may be operable to deliver different categories of drugs (or medications), such as chemotherapy drugs, pain relief drugs, diabetes treatment drugs (e.g., insulin, glucagon and/or glucagon-like peptides), blood pressure medication, or the like.

A type of medication delivery algorithm (MDA) may include an "artificial pancreas" algorithm-based system, or more generally, an artificial pancreas (AP) application. For ease of discussion, the computer programs and computer applications that implement the medication delivery algorithms or applications may be referred to herein as an "AP application." An AP application may be configured to provide automatic delivery of insulin based on an analyte sensor input, such as signals received from an analyte sensor, such as a continuous blood glucose monitor, or the like. In an example, the artificial pancreas (AP) application when executed by a processor may enable monitoring of a user's blood glucose measurement values, determine an appropriate level of insulin for the user based on the monitored glucose values (e.g., blood glucose concentrations or blood glucose measurement values) and other information, such as information related to, for example, a correction factor or substance sensitivity factor, and take actions to maintain a user's blood glucose value within an appropriate range. "Insulin adjustment factor" may refer to a value that is used to adjust or modify the dosage of the therapeutic exogenous substance to offset or account for an effect of endogenous production of endogenous insulin related to the therapeutic exogenous substance. "Substance sensitivity factor" may refer to a value that quantifies a user's ability to metabolize a therapeutic exogenous substance. "Therapeutic exogenous substance" may refer to a substance that is input into the body of the user subcutaneously, intraperitoneally, or intravenously. Examples include insulin, glucagon, glucagon-like peptides, chemotherapy drugs, high blood pressure medications, fertility medications, pain medications, hormones, and the like. "Endogenous insulin" may refer to insulin that is produced in a user's body (i.e., endogenously). A target analyte measurement setting, such as a target blood glucose measurement value setting of the particular user may alternatively be a range of blood glucose measurement values that are appropriate for the particular user. "Target analyte measurement setting" may refer to a value or a range of values that are appropriate for a user's condition, such as approximately 70 mg/dL to approximately 110 mg/dL for diabetes, which the automatic drug delivery system attempts to maintain through management of deliveries of therapeutic exogenous substances, such as insulin and the like. For example, a target blood glucose measurement value may be acceptable if it falls within the range of 80 mg/dL to 120 mg/dL, which is a range satisfying a clinical standard of care for treatment of diabetes. In addition, an AP application as described herein may determine when a user's blood glucose wanders into the hypoglycemic range (e.g., below 70 mg/dL) or the hyperglycemic range (e.g., above 180 mg/dL).

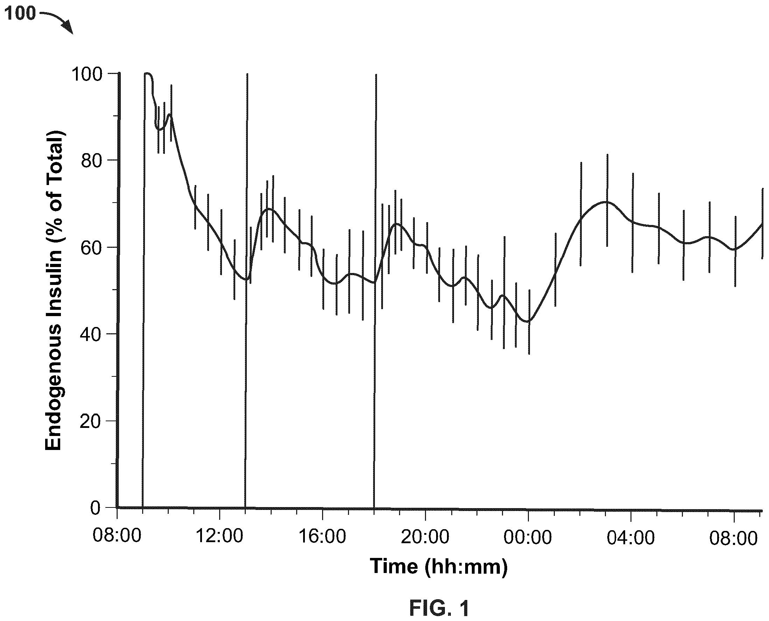

As described in more detail with reference to the examples of FIGs. 1-5, an automatic drug delivery system may be configured to deliver compensating doses of a therapeutic exogenous substance that account for production of endogenous insulin that is related to the therapeutic exogenous substance. "Compensating dose" may refer to an amount of a therapeutic exogenous substance that has been calculated using a dosage of the therapeutic exogenous substance generated by a medication delivery algorithm and an insulin adjustment factor. For ease of discussion, insulin is referred to as the therapeutic exogenous substance of interest, but the techniques and devices described herein may be used to address a similar problem with other therapeutic substances.

Endogenous insulin production is constantly changing, and prior to the techniques and processes described herein, endogenous delivery of insulin in T2DM patients was not typically accounted for when delivering insulin.

The following examples provide an automatic insulin delivery algorithm or application that provides an estimated dosage of the therapeutic exogenous substance that accounts for the endogenous production of insulin.

FIG. 1 shows in a graph the response of a type 2 diabetic person's endogenous insulin secretion when exogenous insulin was delivered to the user throughout the day. The graph shows that endogenous insulin delivery can be reduced by up to 60% or more compared to a user's peak endogenous insulin delivery values depending on the exogenous insulin deliveries. The three long vertical lines represent times when the user ate a meal. In general, FIG. 1 shows that the endogenous production of insulin of a person with type 2 diabetes decreases in the presence of exogenous insulin. FIG. 1 was obtained from Hovorka, et al. J Clin Endocrinol Metab 2014. Glucose Turnover After Replacement of Usual Therapy by Insulin in Insulin-naive Type 2 Diabetes Subjects, and is presented to better elucidate the problem that is addressed by the disclosed subject matter as discussed in the following paragraphs and illustrated in the accompanying figures.

An automatic drug delivery device (shown in another figure) may include a processor, a reservoir, a pump mechanism and a memory. The reservoir may be configured to contain a therapeutic exogenous substance, such as insulin, glucagon-like peptide, pramlintide, or a combination two or more of them. The pump mechanism may be communicatively coupled to the processor and fluidically coupled to the reservoir. The memory may also be coupled to the processor and be configured to store a substance delivery history and

programming code that, when executed by the processor, configure the drug delivery device to perform processes, techniques and functions, such as those described herein. "Substance delivery history" may refer to a data structure that includes a number of amounts of a substance delivered to the body of a user, delivery times of when respective amounts of the substance was delivered, analyte measurement values corresponding to the delivery times, and the like. Substances include insulin, therapeutic exogenous substances and the like. "Analyte measurement value" may refer to a measurement of an analyte that has been output from the analyte sensor.

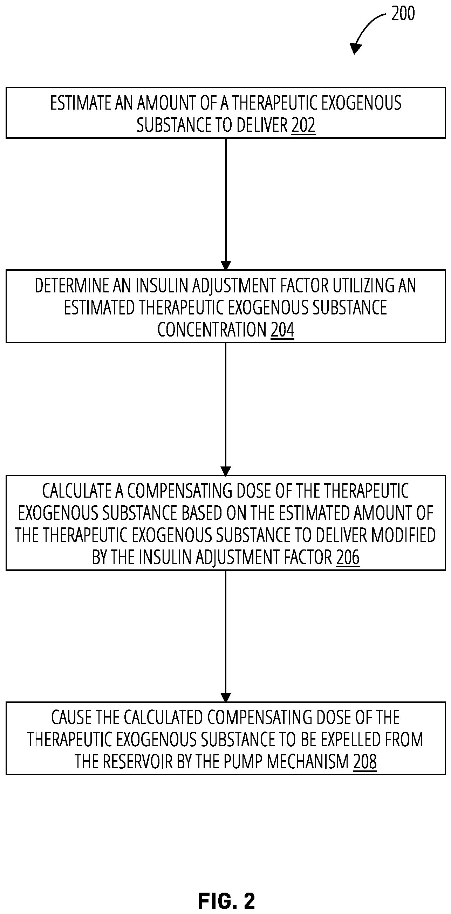

FIG. 2 illustrates a flowchart of an example process according to aspect of the disclosed subject matter.

A processor of an automatic drug delivery device or automatic drug delivery system when executing programming code stored in the memory is configured to implement process 200. In block 202, the processor may be operable to estimate an amount of a therapeutic exogenous substance, such as those discussed above, to deliver to a user. For example, the therapeutic exogenous substance may be insulin, and the processor may execute programming code, such as an MDA or AP application, that makes the processor operable to receive analyte measurement values, such as blood glucose measurement values or the like, from an analyte sensor and use of the analyte measurement values along with other factors, such as historical analyte measurement values, parameters or constraints, in the estimation of the amount of the therapeutic exogenous substance. It may be helpful to describe an example in which insulin is the therapeutic exogenous substance.

In block 204, the processor upon implementing process 200 may determine an insulin adjustment factor utilizing an estimated therapeutic exogenous substance concentration. "Estimated relative therapeutic exogenous substance concentration," which may also be referred to as "relative insulin concentration," may refer to the sum of the past actual therapeutic exogenous substance (e.g., insulin) deliveries over a number of delivery cycles versus the expected insulin needs for the user based on their total daily therapeutic exogenous substance amounts or needs, accounting for the decay and/or absorption of the exogenous insulin within the body over time due to pharmacokinetics or pharmacodynamics of insulin. "Delivery cycle" may refer to a period of time during which different functions are performed by the automatic drug delivery system that may begin with receipt of an analyte measurement value and end with delivery of a dose of a therapeutic exogenous substance, such as a compensating dose of insulin (e.g., that compensates for a high blood glucose level or consumption of a meal). The automatic drug delivery device may also include where the processor is further configured to determine the estimated relative therapeutic exogenous substance concentration (i.e., estimated relative insulin concentration) using a total daily amount of the therapeutic exogenous substance (i.e., insulin) that maintained an analyte measurement value of the user within the range of their target analyte measurement setting.

In an example, the estimated relative insulin concentration may be determined using a number of calculations as shown in the following equation (Eq. 1):

$$I_C = \frac{\sum_{i=1}^{h} I(i)}{h \frac{TDI/24}{12}}$$

where $I_c$ is the estimated relative insulin concentration, $I(i)$ is an amount of therapeutic exogenous substance, in this case insulin, delivered over the period from $i$ to $h$, where $h$ is a tuning factor, and TDI is representative of a total amount of therapeutic exogenous substance delivered in a day, where TDI in the specific example, is the total daily insulin, which is the total amount of insulin delivered over a preset period of time, usually a day (24 hours). The word "relative" in the term "estimated relative insulin concentration ($I_c$)" may refer to the calculation of the insulin delivery that the user has received relative to the user's typical basal delivery. "Tuning factor" may refer to a number of delivery cycles that provide a representative input into a user's compliance with the target analyte measurement setting. The tuning factor $h$ may be a selected number of delivery cycles, such as 2, 3, 6, 9, 12, or the like.

Programming code in the memory executed by the processor may configure the processor to determine the estimated relative insulin concentration $I_c$ by completing Eq 1. In the numerator of Eq. 1, a sum of an amount past actual delivery of the therapeutic exogenous substance over a predetermined number of delivery cycles, where the predetermined number of delivery cycles is indicated by tuning factor $h$. For use in the denominator, the processor may calculate a per cycle delivery amount, i.e.,

$$\frac{TDI/24}{12}$$

(where each cycle is 5 minutes and hence 12 cycles in an hour) by using a total amount of the therapeutic exogenous substance (i.e., TDI) that has been delivered to the user over the course of the preset or predetermined time period (e.g., 24 hours).

Returning to the evaluation of Eq. 1, the processor may apply the tuning factor $h$ to the calculated per cycle delivery amount to obtain a divisor. The sum of an amount past actual delivery of the therapeutic exogenous substance over a predetermined number of delivery cycles is divided by the divisor. The resulting quotient of the dividing is stored in the memory as the estimated relative therapeutic exogenous substance concentration (i.e., $I_c$) of Equation 1.

In an example, the processor may be further configured to determine whether the estimated relative therapeutic exogenous substance concentration $I_c$ has a value within a maximum boundary and a minimum boundary. The maximum boundary may be set at a predetermined percentage of a user's total daily estimated relative therapeutic exogenous substance concentration, which may be a value such as 115%, 130%, 140%, or the like. The percentage when represented as a decimal may be 1.15, 1.30, 1.40, or the like. The minimum boundary may also be a predetermined percentage of a user's total daily estimated relative therapeutic exogenous substance concentration $I_c$ but has a value lower than the maximum boundary. The minimum boundary may be a percentage such as 50%, 65%, 70%, 85%, or the like, which when represented by a decimal value may be 0.50, 0.65, 0.70, 0.85, or the like.

In response to the value of the estimated relative therapeutic exogenous substance concentration being between or within the maximum and minimum boundaries (i.e., less than the maximum boundary (also referred to as a maximum adjustment factor) but greater than the minimum boundary (also referred to as a minimum adjustment factor)), the processor may set the correction factor, although, it is more appropriately referred to as an insulin adjustment factor (i.e., $I_{cfactor}$) to be equal to the estimated relative insulin concentration or the estimated relative therapeutic exogenous substance concentration. For example, the result of estimated relative therapeutic exogenous substance concentration $I_c$ from Eq. 1 above may be approximately 1.1. The processor may be operable to evaluate the estimated relative therapeutic exogenous substance concentration $I_c$ result according to the following equation (Eq. 2) to determine the correction factor:

$$I_{Cfactor} = \max(\min(I_C, 1.3), 0.7)$$

As shown in Eq. 2, the processor of the automatic drug delivery device may further be configured to determine a minimum value between the estimated relative therapeutic exogenous substance concentration or a value of the maximum insulin adjustment factor (i.e., $\min(I_c, 1.3)$). Once the determination of the minimum value between the estimated relative therapeutic exogenous substance concentration or a value of the maximum insulin adjustment factor is made, the processor may further evaluate the determined minimum value.

In an example in which the therapeutic exogenous substance is insulin and the estimated relative therapeutic exogenous substance concentration is an estimated insulin concentration. For example, the estimated relative insulin concentration $I_c$ may be 0.9. In the evaluation of Eq. 2 in such an example with the estimated relative insulin concentration $I_c$ being 0.9, the minimum value between the estimated relative insulin concentration $I_c$ value of 0.9 and 1.3 is the estimated relative insulin concentration $I_c$ value of 0.9. The processor continuing to evaluate Eq. 2 and may further determine a maximum between the minimum value and the minimum boundary, which in the above example of Eq. 2 is 0.7. The processor, in response to the value of the estimated relative insulin concentration being greater than the value of the minimum boundary, set the insulin adjustment factor to be equal to the value of the estimated relative insulin concentration, which in this example is 0.9.

Alternatively, in an example where the estimated relative insulin concentration $I_c$ is 1.6, the processor, when determining the minimum function, may in response to the value of the estimated relative insulin concentration $I_c$ (i.e., 1.6) being greater than the maximum boundary (i.e., 1.3), the processor may set the insulin adjustment factor $I_{cfactor}$ to be equal to the value of the maximum insulin adjustment factor, such as 1.3 in this case. This is evident by working through the maximum and minimum functions in Eq. 2. With the estimated relative insulin concentration $I_c$ value being 1.6 in this example, the minimum function returns a value of 1.3 and the maximum function which evaluates the values 1.3 and 0.7 returns the 1.3 as the setting for the $I_{cfactor}$ of Eq. 2.

Or, in a further alternative of the evaluation of Eq. 2, the estimated relative insulin concentration may be 0.5. The processor, in response to the value of the estimated relative therapeutic exogenous substance concentration (i.e, the estimated relative insulin concentration of 0.5) being less than the value of the minimum boundary (e.g., 0.7 in Eq. 2), may set the insulin adjustment factor $I_{cfactor}$ to be equal to a value of an estimated relative insulin concentration, which is 0.5 in this example.

In block 206, the processor may calculate a compensating dose of the therapeutic exogenous substance based on the estimated amount of the therapeutic exogenous substance to deliver either based on or modified using the insulin adjustment factor $I_{cfactor}$. For example, the processor may calculate the compensating dose according to the following equation (Eq. 3):

$$I_{final} = I_{AID} \cdot I_{Cfactor}$$

where $I_{AID}$ is the estimated amount of the therapeutic exogenous substance to deliver, $I_{cfactor}$ is the insulin adjustment factor, and $I_{final}$ is the compensating dose.

The compensating dose may be calculated to counteract an effect of reduced production of an endogenous insulin that is the compliment to the therapeutic exogenous substance. In more detail, the delivery of amounts of the therapeutic exogenous substance may cause the user's body to produce less of the endogenous insulin because the body reacts to the presence of the therapeutic exogenous substance. As a result, the processor when calculating the compensating dose of the therapeutic exogenous substance may be configured to modify the estimated amount of the therapeutic exogenous substance using the insulin adjustment factor.

In a further example, a memory coupled to the processor may be configured to store a substance delivery history. The substance delivery history may be a record of the doses of the therapeutic exogenous substance, including previous compensating doses, that have been delivered to the user over a period of time. The period of time may be a number of hours, days, weeks, or months, or a number of delivery cycles, such as 60, 120, 240, or the like. In the further example, the processor may obtain, from the substance delivery history stored in the memory, a total amount of the therapeutic exogenous substance that has been delivered to a user over the course of a predetermined time period. Based on the obtained total amount of the therapeutic exogenous substance, the processor may determine a total daily amount of the therapeutic exogenous substance that maintains the user within range of a target analyte measurement setting, such as 70 to 150 mg/dL or the like.

The processor may be communicatively coupled to a pump mechanism. The automatic drug delivery system may, in addition to including the processor and pump mechanism include a reservoir. The reservoir may be configured to contain the therapeutic exogenous substance. The pump mechanism may be operable to expel the therapeutic exogenous substance in response to commands from the processor. In block 208, the processor executing process 200 may cause a pump mechanism to expel the calculated compensating dose of the therapeutic exogenous substance from a reservoir.

In addition, or alternatively, the insulin adjustment factor $I_{cfactor}$ can be incorporated as a multiplier to the Type-2 diabetic's *open loop* insulin deliveries as well. For example, the user may determine a dosage of insulin to deliver and the insulin adjustment factor may be applied according the above described processes

In the following examples, the calculated compensating dose may be limited to a maximum level to mitigate increases that may be due primarily to feedback in the process 200. The processor executing process 200 may cause the processor when calculating the compensating dose of the therapeutic exogenous substance to modify the estimated amount of the therapeutic exogenous substance using the insulin adjustment factor $I_{cfactor}$.

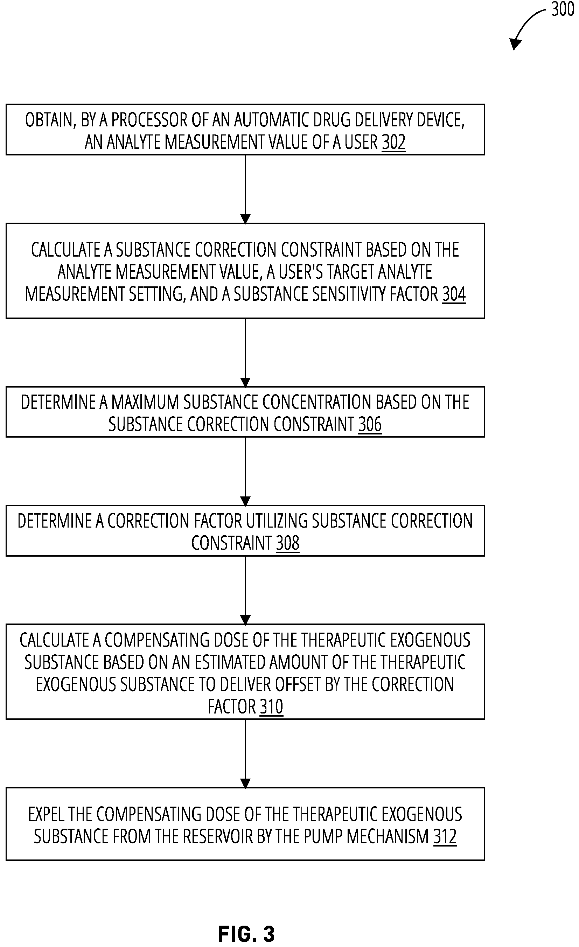

FIG. 3 illustrates a flowchart of another example process according to another exemplary aspect of the disclosed subject matter. In an example, a processor of an automatic drug delivery device executing programming code may be configured to implement process 300. In block 302, the processor of the automatic drug delivery device may obtain an analyte measurement value (e.g., a blood glucose measurement value at time t, or EGV(t)) of a user. The processor may receive the analyte measurement value from an analyte sensor that is communicatively coupled to the processor. Alternatively, the processor may retrieve the analyte measurement value from a memory that is communicatively coupled to the processor. In some examples, the analyte measurement value retrieved from the memory may be an estimate of the analyte measurement value based on previous analyte measurement values received from the analyte sensor.

In block 304, the processor may be configured to calculate a substance correction constraint ($I_{corr}$) based on the analyte measurement value (EGV(t)), a user's target analyte measurement setting (e.g., the user's set point at time t, or SP(t)), and a substance sensitivity factor (which may be calculated by 1800/TDI). A "substance correction constraint" may refer to a value that is indicative of how closely a user's analyte measurement values are to remaining within a threshold range of the user's target analyte measurement setting. For example, the processor may determine the substance correction constraint ($I_{corr}$) by evaluating an equation such as Eq. 3, as follows:

$$I_{corr} = \frac{EGV(t) - SP(t)}{\frac{1800}{TDI}}$$

For example, the processor may determine a difference between the analyte measurement value (EGV(t)) and a target analyte measurement setting (SP(t)) of a user of the automatic drug delivery device. The processor may divide the difference (EGV(t)-SP(t)) by the substance sensitivity factor (1800/TDI), wherein a quotient resulting from the dividing is the substance correction constraint ($I_{corr}$).

In block 306, a processor implementing process 300 may determine a maximum substance concentration ($I_{C,Max}$) based on the substance correction constraint. "Maximum substance concentration ($I_{C,Max}$)" may refer to a maximum setting for the substance concentration based on a number of factors including amount of the substance onboard and the substance correction constraint. For example, the maximum substance concentration ($I_{C,Max}$) may be determined using an equation such as Eq. 4, as follows:

$$I_{C,Max} = 1 + 0.3 \min\left(1, \frac{I_{corr}}{\max\left(I_{corr}, IOB(t)\right)}\right)$$

In the example of Equation 4, the processor may determine a maximum ($\max(I_{corr}, IOB(t))$) between the substance correction constraint ($I_{corr}$) and a substance on board parameter (e.g., insulin on board or within the body at time t, or IOB(t)). "A substance on board parameter" may refer to an amount of a substance, such as insulin in the body of a user. The substance on board parameter accounts for amounts of a therapeutic exogenous substance that may have not yet been metabolized by the user's body. The processor may further divide the substance correction constraint ($I_{corr}$) by the determined maximum ($\max(I_{corr}, IOB(t))$). The processor may be further operable to determine a minimum between a constant term (e.g., one (1)) and a quotient of the division of the substance correction constraint by the determined maximum and multiply determined minimum by a predetermined factor (e.g., 0.3). The value 1, in the example, is considered a primary value and may be representative of 100 percent of $I_{C,Max}$. The processor may add the primary value (which in this case is 1, but may be 0.9, 0.5, or the like) to the result of the multiplying of the determined minimum by a predetermined factor to obtain the maximum substance concentration ($I_{C,Max}$). In an example of a Type-2 diabetic, Eq. 4 indicates that the originally stated maximum threshold, 1.3, can be adjusted down to 1 if there is already sufficient insulin delivered or the user's glucose concentration is close to or below the setpoint.

In block 308, process 300 determines an insulin adjustment factor ($I_{cfactor}$) utilizing substance correction constraint. The insulin adjustment factor $I_{cfactor}$ may be determined using an equation such as Eq. 5:

$$I_{Cfactor} = \max\big(\min\big(I_C, I_{C,max}\big), 0.7\big)$$

In determining the insulin adjustment factor, the processor may obtain the maximum substance concentration ($I_{C,Max}$) that was calculated using the substance correction constraint ($I_{corr}$). The processor may determine a minimum between estimated relative insulin concentration ($I_C$) and the maximum substance concentration ($I_{C,Max}$). A maximum ($\max(\min(I_C, I_{C,Max}), 0.7)$) of the determined minimum between $I_C$ and $I_{C,Max}$ and a lower correction boundary (e.g., 0.7) may be determined. The processor may set the determined maximum as the insulin adjustment factor $I_{cfactor}$.

In block 310, process 300 calculates a compensating dose of the therapeutic exogenous substance based on an estimated amount of the therapeutic exogenous substance to deliver, adjusted by the insulin adjustment factor $I_{cfactor}$. The compensating dose $I_{final}$ may, for example, be calculated using Eq. 3 from above, which is:

$$I_{final} = I_{AID} \cdot I_{Cfactor}$$

In block 312, a control signal may be generated by the processor in completion of the process 300 that causes the pump mechanism to expel the compensating dose ($I_{final}$) of the therapeutic exogenous substance from the reservoir. The control signal may contain information, such as the compensating dose, a timing for delivery of the compensating dose, and the like.

In the automatic insulin delivery algorithm or application, the estimated insulin concentration $I_C$ may increase due to significant periods of elevated therapeutic delivery prior to a current cycle, thus in turn increase current therapeutic delivery of insulin, up to a physiological limit for reduction in endogenous insulin deliveries.

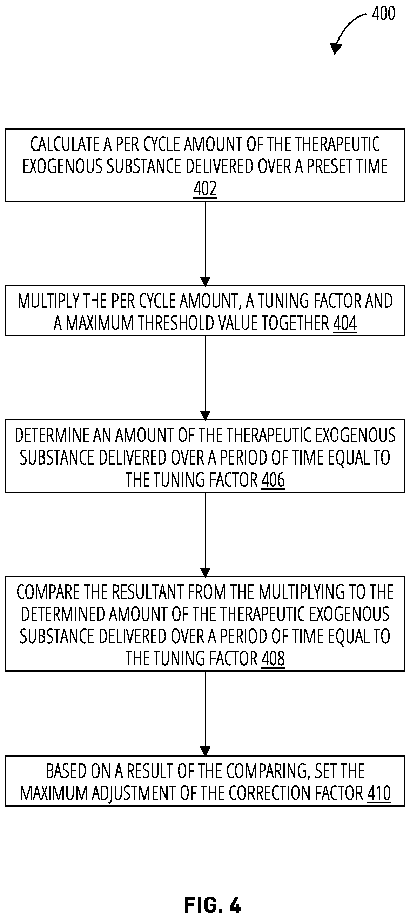

FIG. 4 illustrates a flowchart of an example of a detailed process according to another exemplary aspect of the disclosed subject matter. In the example of FIG. 4, the therapeutic exogenous substance may be insulin. As an alternative embodiment, a maximum adjustment of the insulin delivery factor can be limited to never exceed a certain proportion of the user's TDI as shown in equation 6 (Eq. 6) below. Specifically, the insulin adjustment factor $I_{cfactor}$ can be further limited by reviewing the sum of the user's past insulin deliveries versus the threshold of 30% (e.g., 1.30) higher than the estimated total delivery based on the user's TDI. Namely, this can be considered as a "constraint" on the value of the estimated relative insulin concentration $I_c$. This also reduces the risk of overdelivery in case of large manual boluses by automatically limiting the insulin adjustment factor $I_{cfactor}$ from behaving more aggressively than desired until $h$ cycles have passed.

In this example described with reference to FIG. 4, the maximum adjustment of the insulin adjustment factor $I_{cfactor}$ can be bounded based on the estimated endogenous insulin delivery values. Specifically, although the endogenous insulin delivery values are reduced by exogenous insulin delivery, endogenous insulin delivery values may also be increased based on the user's current glucose concentrations due to the natural feedback of the user's endocrine system. Consequently, any increases in insulin delivery due to the insulin adjustment factor $I_{cfactor}$ can be applied only up to insulin requests that may not exceed the endogenous insulin delivery that would have occurred without the antagonistic effects of the exogenous insulin delivery.

The processor may implement process 400 to determine the maximum adjustment for the insulin adjustment factor $I_{cfactor}$ to a final insulin adjustment factor $I_{c,final}$ using the following (Eq. 6):

$$I_{c,final} = \begin{cases} I_{Cfactor} & \sum_{i=1}^{h} I(i) < 1.3 \cdot h \cdot \dfrac{TDI}{288} \\ 1 & \sum_{i=1}^{h} I(i) \geq 1.3 \cdot h \cdot \dfrac{TDI}{288} \end{cases}$$

In the implementation of the process 400, the processor may obtain, for example, from a substance delivery history stored in a memory, a total amount of a therapeutic exogenous substance that has been delivered to a user over a preset time period.

During execution of process 400 at block 402, the processor may calculate a per cycle amount of the therapeutic exogenous substance delivered over a preset or predetermined time. In Eq. 6, TDI represents the total amount of the therapeutic exogenous substance that has been delivered to the user over the preset time period. The preset time period may be one day, 24 hours, 36 hours, or the like. The constant value of 288 represents the number of cycles that occur in 24 hour period when a delivery cycle is 5 minutes. When the delivery cycle is 5 minutes, there are 12 cycles in 1 hour (60 minutes) and 288 cycles in 24 hours. Of course, if the preset time period changes from 1 day or 24 hours, the constant value changes from 288 accordingly. The per cycle amount of the therapeutic exogenous substance, is determined by dividing the TDI by the constant value 288.

In block 404, the processor may multiply the per cycle amount, a tuning factor $h$ and a maximum threshold value (e.g., 1.3) together.

Process 400 in block 406 may cause the processor to determine an amount of the therapeutic exogenous substance delivered over a period of time equal to the tuning factor, which is a sum of an amount past actual delivery of the therapeutic exogenous substance over a period of time corresponding to the tuning factor $h$. The period of time, for example, may be measured from $i$ to $h$, where $i$ = zero or one, and $h$ is a number of delivery cycles. The delivery cycles, in this example, are 5-minute increments.

At block 408, the result from the multiplying (of the per cycle amount, a tuning factor $h$ and a maximum threshold value (e.g., 1.3)) is compared to the determined amount of the therapeutic exogenous substance delivered over a period of time equal to the tuning factor $h$.

In block 410, based on a result of the comparing, the processor may set the maximum adjustment of the insulin adjustment factor by providing a final insulin adjustment factor $I_{cfinal}$. For example, the processor in response to a result of the comparing indicating that the result is less than or equal to the determined amount of the therapeutic exogenous substance delivered over a period of time equal to the tuning factor, may set the final insulin adjustment factor to one. Alternatively, the processor in response to result of the comparing indicating that the result (of 1.3 x $h$ x (TDI/288)) is greater than the determined amount of the therapeutic exogenous substance delivered over a period of time from $i$ to the tuning factor $h$, may set the final insulin adjustment factor $I_{c,final}$ as the insulin adjustment factor $I_{cfactor}$. The insulin adjustment factor $I_{cfactor}$ was initially determined to account for a decrease in the user's body's production of the endogenous insulin as a result of the therapeutic exogenous substance administered by the automatic drug delivery device.

It may be helpful to understand the implementation of the various techniques and processes to review an example of a system suitable for implementing the techniques and processes described with reference to FIGs. 2-4.

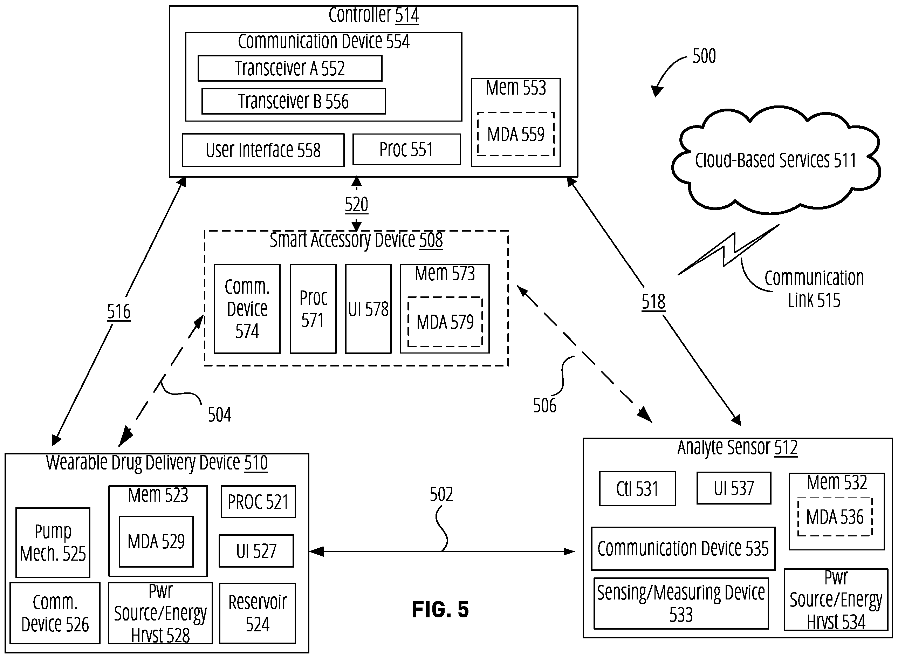

FIG. 5 illustrates a functional block diagram of a system example suitable for implementing the example processes and techniques described herein.

The automatic drug delivery system 500 may implement (and/or provide functionality for) a medication delivery algorithm (MDA), such as an artificial pancreas (AP) application, to govern or control automated delivery of a drug or medication, such as insulin, to a user (e.g., to maintain euglycemia - a normal level of glucose in the blood). The drug delivery system 500 may be an automated drug delivery system that may include a wearable automatic drug delivery device 510, an analyte sensor 512, and a controller 514.

The system 500, in an optional example, may also include a smart accessory device 508, such as a smartwatch, a personal assistant device or the like, which may communicate with the other components of system 500 via either a wired or wireless communication links 504, 506 and 520.

The controller 514 may be a computing device such as a smart phone, a tablet, a personal diabetes controller, a dedicated diabetes therapy controller, or the like. In an example, the controller 514 may include a processor 551, a controller memory 553, a user interface 558, and a communication device 554. The controller 514 may contain analog and/or digital circuitry that may be implemented as a processor 551 for executing processes based on programming code stored in the controller memory 553, such as the medication delivery algorithm or application (MDA) 559, to manage a user's blood glucose levels and for controlling the delivery of the drug, medication, or therapeutic agent to the user as well as other functions, such as calculating carbohydrate-compensation dosage, a correction bolus dosage and the like as discussed above. The controller 514 may be used to program, adjust settings, and/or control operation of the wearable automatic drug delivery device 510 and/or the analyte sensor 512 as well as the optional smart accessory device 508.

The processor 551 may also be configured to execute programming code stored in the controller memory 553, such as the MDA 559. The MDA 559 may be a computer application that is operable to deliver a drug based on information received from the analyte sensor 512, the cloud-based services 511 and/or the controller 514 or optional smart accessory device 508. The memory 553 may also store programming code to, for example, operate the user interface 558 (e.g., a touchscreen device, a camera or the like), the communication device 554 and the like. The processor 551 when executing the MDA 559 may be configured to implement techniques and processes related to determining insulin adjustment factors, estimated relative therapeutic exogenous substance concentrations or the estimated relative insulin concentrations, maximum and minimum values, tuning (or tunable) factors, control signals, and the like. The user interface 558 may be under the control of the processor 551 and be configured to present a graphical user interface that enables the input and output of blood glucose target settings, tuning factors, bolus doses, a selection of whether the user is a Type 1 or a Type 2 diabetic, and the like as described above. For example, in response to an indication the user is a Type 2 diabetic, the processes of FIGs. 2-4 may be implemented by a respective instance of the MDA, such as 559, 529, 579 or 536.

In a specific example, when the MDA 559 is an artificial pancreas (AP) application, the processor 551 is also configured to execute a diabetes treatment plan (which may be stored in a memory) that is managed by the MDA 559 stored in memory 553. In addition to the functions mentioned above, when the MDA 559 is an AP application, it may further provide functionality to enable the processor 551 to determine a dosage of a therapeutic exogenous substance, such as insulin, based on prior dosages of the therapeutic exogenous substance administered by the drug delivery device 510 and the like according to a diabetes treatment plan. In addition, as an AP application, the MDA 559 provides functionality to enable the processor 551 to output signals to the wearable automatic drug delivery device 510 to deliver the determined bolus and basal dosages described with reference to the examples of FIGs. 2-4.

The communication device 554 may include one or more transceivers such as Transceiver A 552 and Transceiver B 556 and receivers or transmitters that operate according to one or more radio-frequency protocols. In the example, the transceivers 552 and 556 may be a cellular transceiver and a Bluetooth® transceiver, respectively. For example, the transceiver A 552 or transceiver B 556 may be configured to receive and transmit signals containing information usable by the MDA 559.

The controller 514 may include a user interface 558, which may be a keypad, a touchscreen display, levers, light-emitting diodes, buttons on a housing of the controller 514, a microphone, a camera, a speaker, a display, or the like, that is configured to allow a user to enter information and allow the controller 514 to output information for presentation to the user (e.g., alarm signals or the like). The user interface 558 may provide inputs, such as a voice input, a gesture (e.g., hand or facial) input to a camera, swipes to a touchscreen, or the like, to processor 551 which the programming code interprets.

The wearable automatic drug delivery device 510, in the example system 500, may include a user interface 527, a processor 521, a pump mechanism 525, a communication device 526, a memory 523, a power source 528, and a reservoir 524. The wearable automatic drug delivery device 510 may be configured to perform and execute the processes described in the examples of FIGs. 2-4 without input from the controller 514 or the optional smart accessory device 508. As explained in more detail, the processor 521 may be operable, for example, implement the processes of FIGs. 2-4 as well as determine an amount of insulin delivered, IOB, insulin remaining, and the like. The processor 521 alone may implement the processes of FIGs. 2-4 as well as determine an amount of insulin delivered, IOB, insulin remaining, and the like, such as control insulin delivery, based on an input from the analyte sensor 504.

The memory 523 may store programming code executable by the processor 521. The programming code, for example, may enable the processor 521 to control expelling insulin from the reservoir 524 and control the administering of doses of medication based on signals from the MDA 529 or, external devices, if the MDA 529 is configured to implement the external control signals.

The reservoir 524 may be configured to store drugs, medications or therapeutic agents suitable for automated delivery, such as diabetes treatment drugs (e.g., insulin, glucagon, glucagon-like peptides), pain relief drugs (e.g., morphine), hormones, blood pressure medicines, chemotherapy drugs, or the like.

In an example, the wearable automatic drug delivery device 510 includes a communication device 526, which may be a receiver, a transmitter, or a transceiver that operates according to one or more radio-frequency protocols, such as Bluetooth, Wi-Fi, a near-field communication standard, a cellular standard, or the like. The processor 521 may, for example, communicate with the personal diabetes controller 514 and the analyte sensor 512 via the communication device 526.

The wearable automatic drug delivery device 510 may be attached to the body of a user, such as a patient or diabetic, at an attachment location and may deliver any therapeutic exogenous substance, including any drug, therapeutic, or medicine, such as insulin, glucagon, glucagon-like peptide, or the like, to a user at or around the attachment location. A surface of the wearable automatic drug delivery device 510 may include an adhesive to facilitate attachment to the skin of a user as described in earlier examples.

The wearable automatic drug delivery device 510 may, for example, include a reservoir 524 for storing the drug (such as insulin), a needle or cannula (not shown in this example) for delivering the drug into the body of the user (which may be done subcutaneously, intraperitoneally, or intravenously), and a pump mechanism 525 for transferring the drug from the reservoir 524 through a needle or cannula and into the user. The pump mechanism 525 may be fluidly coupled to reservoir 524, and communicatively coupled to the processor 521.

The wearable automatic drug delivery device 510 may further include a power source 528, such as a battery, a piezoelectric device, other forms of energy harvesting devices, or the like, for supplying electrical power to the pump mechanism 525 and/or other components (such as the processor 521, memory 523, and the communication device 526) of the wearable automatic drug delivery device 510.

In some examples, the wearable automatic drug delivery device 510 may include a user interface 527, which may be a keypad, a touchscreen display, levers, light-emitting diodes, buttons on a housing of the drug delivery device 510, a microphone, a camera, a speaker, a display, or the like, that is configured to allow a user to enter information and allow the drug delivery device 510 to output information for presentation to the user (e.g., alarm signals or the like). The user interface 527 may provide inputs, such as a voice input, a gesture (e.g., hand or facial) input to an optical sensor, swipes to a touchscreen, or the like, to processor 521 which the programming code interprets.

When configured to communicate to an external device, such as the controller 514 or the analyte sensor 504, the wearable automatic drug delivery device 510 may receive signals over the wired or wireless link 594 from the controller 514 or 508 from the analyte sensor 504. The processor 521 of the wearable automatic drug delivery device 510 may receive and process the signals from the respective external devices as described with reference to the examples of FIGs. 2-4 as well as implementing delivery of a drug to the user according to a diabetes treatment plan or other drug delivery regimen.

In an operational example, the processor 521 when executing the MDA 529 may output a control signal operable to actuate the pump mechanism 525 to deliver a compensation dosage of insulin, a bolus, a revised basal dosage or the like as described with reference to the examples of FIGs 2-4.

The smart accessory device 508 may be, for example, a smart watch, another wearable smart device, including eyeglasses, provided by other manufacturers, a global positioning system-enabled wearable, a wearable fitness device, smart clothing, or the like. Similar to the controller 514, the smart accessory device 508 may also be configured to perform various functions including controlling the wearable automatic drug delivery device 510. For example, the smart accessory device 508 may include a communication device 574, a processor 571, a user interface 578 and a memory 573. The user interface 578 may be a graphical user interface presented on a touchscreen display of the smart accessory device 508. The memory 573 may store programming code to operate different functions of the smart accessory device 508 as well as an instance of the MDA 579. The processor 571 that may execute programming code, such as MDA 579 for controlling the wearable automatic drug delivery device 510 to implement the processes and techniques of FIG. 2-4 described herein.

The analyte sensor 512 may include a controller 531, a memory 532, a sensing/measuring device 533, a user interface 537, a power source/energy harvesting circuitry 534, and a communication device 535. The analyte sensor 512 may be communicatively coupled to the processor 551 of the controller 514 or processor 521 of the wearable automatic drug delivery device 510. The memory 532 may be configured to store information and programming code, such as an instance of the MDA 536.

The analyte sensor 512 may be configured to detect multiple different analytes, such as lactate, ketones, uric acid, sodium, potassium, alcohol levels, blood glucose, proteins, hormones, or the like, and output results of the detections, such as measurement values or the like. The analyte sensor 512 may, in an example, be configured to measure the blood glucose value at a predetermined time interval, such as every 5 minutes, or the like. The communication device 535 of analyte sensor 512 may have circuitry that operates as a transceiver for communicating the measured blood glucose values to the controller 514 over a wireless link 515 or with wearable automatic drug delivery device 510 over the wireless communication link 502. While called an analyte sensor 512, the sensing/measuring device 533 of the analyte sensor 512 may include one or more additional sensing elements, such as a heart rate monitor, a pressure sensor, or the like. The controller 531 may include discrete, specialized logic and/or components, an application-specific integrated circuit, a microcontroller or processor that executes software instructions, firmware, programming instructions stored in memory (such as memory 532), or any combination thereof.

Similar to the processor 521, the controller 531 of the analyte sensor 512 may be operable to perform many functions. For example, the controller 531 may be configured by the programming code stored in the memory 532 to manage the collection and analysis of data detected the sensing and measuring device 533, such as blood glucose measurement values, providing trend information and the like.

Although the analyte sensor 512 is depicted in FIG. 5 as separate from the wearable automatic drug delivery device 510, in various examples, the analyte sensor 512 and wearable automatic drug delivery device 510 may be incorporated into the same unit. That is, in various examples, the sensor 512 may be a part of the wearable automatic drug delivery device 510 and contained within the same housing of the wearable automatic drug delivery device 510 (e.g., the sensor 512 or, only the sensing/measuring device 533 and memory storing related programming code may be positioned within or integrated into, or into one or more components, such as the memory 523, of, the wearable automatic drug delivery device 510). In such an example configuration, the processor 521 may be able to implement the process examples of FIGs. 2-4 alone without any external inputs from the controller 514, the cloud-based services 511, another sensor (not shown), the optional smart accessory device 508, or the like.

The communication link 515 that couples the cloud-based services 511 to the respective devices 510, 512, 514 or 508 of system 500 may be a cellular link, a Wi-Fi link, a Bluetooth link, or a combination thereof. Services provided by cloud-based services 511 may include data storage that stores anonymized data, such as blood glucose measurement values, historical IOB or TDI, maximum and minimum boundary values, therapeutic exogenous substance concentration values, substance sensitivity values, and other forms of data. In addition, the cloud-based services 511 may process the anonymized data from multiple users to provide generalized information related to TDI, insulin sensitivity, insulin onboard (IOB), and the like.

The wireless communication links 502, 504, 506, 516, 518, and 520 may be any type of wireless link operating using known wireless communication standards or proprietary standards. As an example, the wireless communication links 502, 504, 506, 516, 518, and 520 may provide communication links based on Bluetooth®, Zigbee®, Wi-Fi, a near-field communication standard, a cellular standard, or any other wireless protocol via the respective communication devices 508, 510, 512, and 514.

Software related implementations of the techniques described herein, such as the processes examples described with reference to FIGs. 2-4 may include, but are not limited to, firmware, application specific software, or any other type of computer readable instructions that may be executed by one or more processors. The computer readable instructions may be provided via non-transitory computer-readable media. Hardware related implementations of the techniques described herein may include, but are not limited to, integrated circuits (ICs), application specific ICs (ASICs), field programmable arrays (FPGAs), and/or programmable logic devices (PLDs). In some examples, the techniques described herein, and/or any system or constituent component described herein may be implemented with a processor executing computer readable instructions stored on one or more memory components.

In addition, or alternatively, while the examples may have been described with reference to a closed loop algorithmic implementation, variations of the disclosed examples may be implemented to enable open loop use. The open loop implementations allow for use of different modalities of delivery of insulin such as smart pen, syringe or the like. For example, the disclosed AP application and algorithms may be operable to perform various functions related to open loop operations, such as the generation of prompts requesting the input of information such as weight or age. Similarly, a dosage amount of insulin may be received by the AP application or algorithm from a user via a user interface. Other open-loop actions may also be implemented by adjusting user settings or the like in an AP application or algorithm.

Some examples of the disclosed device or processes may be implemented, for example, using a storage medium, a computer-readable medium, or an article of manufacture which may store an instruction or a set of instructions that, if executed by a machine (i.e., processor or controller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD), a tape, a cassette, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the spirit and scope of the disclosed examples. In fact, variations, modifications, and other implementations of what was described herein will occur to those of ordinary skill in the art without departing from the spirit and the scope of the disclosed examples. As such, the disclosed examples are not to be defined only by the preceding illustrative description.

Program aspects of the technology may be thought of as "products" or "articles of manufacture" typically in the form of executable code and/or associated data that is carried on or embodied in a type of non-transitory, machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, inventive subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of examples has been presented for the purposes of illustration and description. It is not intended to be exhaustive or to limit the present disclosure to the precise forms disclosed. Many modifications and variations are possible in light of this disclosure. It is intended that the scope of the present disclosure be limited not by this detailed description, but rather by the claims appended hereto. Future filed applications claiming priority to this application may claim the disclosed subject matter in a different manner and may generally include any set of one or more limitations as variously disclosed or otherwise demonstrated herein.

Although the present invention is defined in the attached claims, it should be understood that the present invention can also (alternatively) be defined in accordance with the following embodiments:

1. An automatic drug delivery device, comprising:

a processor,

a reservoir configured to contain a therapeutic exogenous substance;

a pump mechanism communicatively coupled to the processor, and fluidically coupled to the reservoir; and

a memory coupled to the processor and configured to store a substance delivery history and programming code that, when executed by the processor, cause the drug delivery device to: estimate an amount of the therapeutic exogenous substance to deliver to a user;

determine an insulin adjustment factor utilizing an estimated relative therapeutic exogenous substance concentration;

calculate a compensating dose of the therapeutic exogenous substance based on the estimated amount of the therapeutic exogenous substance to deliver that is adjusted by the insulin adjustment factor; and

causing the calculated compensating dose of the therapeutic exogenous substance to be expelled from the reservoir by the pump mechanism.

2. The automatic drug delivery device of embodiment 1, wherein the processor when calculating the compensating dose of the therapeutic exogenous substance is further configured to:
modify the estimated amount of the therapeutic exogenous substance using the insulin adjustment factor.

3. The automatic drug delivery device of embodiment 1, wherein the processor when calculating the compensating dose of the therapeutic exogenous substance is further configured to:

obtain, from the substance delivery history stored in the memory, a total amount of the therapeutic exogenous substance that has been delivered to the user over a preset time period; and

determine, based on the obtained total amount of the therapeutic exogenous substance, a total daily amount of the therapeutic exogenous substance that maintains the user within range of a target analyte measurement setting.

4. The automatic drug delivery device of embodiment 1, wherein the processor, when determining the insulin adjustment factor utilizing the estimated relative therapeutic exogenous substance concentration, is further configured to:

determine whether the estimated relative therapeutic exogenous substance concentration has a value within a maximum boundary and a minimum boundary; and
in response to the value of the estimated relative therapeutic exogenous substance concentration being within the maximum boundary and the minimum boundary, set the insulin adjustment factor to be equal to the therapeutic exogenous substance concentration.

5. The automatic drug delivery device of embodiment 1, wherein the processor is further configured to:
determine the estimated relative therapeutic exogenous substance concentration using a total daily amount of the therapeutic exogenous substance that maintained an analyte measurement value of the user within a range of a target analyte measurement setting.

6. The automatic drug delivery device of embodiment 1, wherein the processor is further configured to:

determine a sum of an amount past actual delivery of the therapeutic exogenous substance over a predetermined number of delivery cycles;

calculate a per cycle delivery amount of using a total amount of the therapeutic exogenous substance that has delivered to the user over a preset time period;

apply a tuning factor that corresponds to the predetermined number of delivery cycles to the calculated per cycle delivery amount to obtain a divisor;

divide the sum of an amount past actual delivery of the therapeutic exogenous substance over a predetermined number of delivery cycles by the obtained divisor; and

store a quotient of the dividing that is the estimated relative therapeutic exogenous substance concentration.

7. The automatic drug delivery device of embodiment 6, wherein the processor is further configured to:

determine a minimum value between the estimated relative therapeutic exogenous substance concentration or a maximum insulin adjustment factor;

based on the determined minimum, determine a maximum value between the estimated relative therapeutic exogenous substance concentration and a minimum boundary; and

in response to a value of the estimated relative therapeutic exogenous substance concentration being greater than the minimum boundary, set the insulin adjustment factor to be equal to the value of the estimated relative therapeutic exogenous substance concentration.

8. The automatic drug delivery device of embodiment 6, wherein the processor is further configured to:

determine whether the estimated relative therapeutic exogenous substance concentration has a value within a maximum boundary and a minimum boundary; and
in response to the value of the estimated relative therapeutic exogenous substance concentration being greater than the maximum boundary, set the insulin adjustment factor to be equal to a maximum insulin adjustment factor.

9. The automatic drug delivery device of embodiment 6, wherein the processor is further configured to:

determine whether the estimated relative therapeutic exogenous substance concentration has a value within a maximum boundary and a minimum boundary; and
in response to the value of the estimated relative therapeutic exogenous substance concentration being less than the minimum boundary, set the insulin adjustment factor to be equal to the minimum boundary.

10. The automatic drug delivery device of embodiment 1, wherein the processor is further configured to:
determine a maximum adjustment of the insulin adjustment factor.

11. The automatic drug delivery device of embodiment 10, wherein the processor, when determining the maximum adjustment of the insulin adjustment factor is further configured to:

calculate a per cycle amount of the therapeutic exogenous substance delivered over a preset time;
multiply the per cycle amount, a tuning factor and a maximum threshold value together;
determine an amount of the therapeutic exogenous substance delivered over a period of time equal to the tuning factor;
compare a result from the multiplying to the determined amount of the therapeutic exogenous substance delivered over the period of time equal to the tuning factor; and
based on a result of the comparing, set the maximum adjustment of the insulin adjustment factor.

12. The automatic drug delivery device of embodiment 11, wherein setting the maximum adjustment of the insulin adjustment factor further comprises:
in response to result of the comparing indicating that the result is greater than the determined amount of the therapeutic exogenous substance delivered over the period of time equal to the tuning factor, set the insulin adjustment factor as determined to account for production of an endogenous insulin related to the therapeutic exogenous substance administered by the automatic drug delivery device.

13. The automatic drug delivery device of embodiment 11, wherein setting the maximum adjustment of the insulin adjustment factor further comprises:
in response to result of the comparing indicating that the result is less than or equal to the determined amount of the therapeutic exogenous substance delivered over a period of time equal to the tuning factor, set the insulin adjustment factor to one.

14. A method, comprising:

estimating, by a processor of an automatic drug delivery device, an amount of a therapeutic exogenous substance to deliver;

determining an insulin adjustment factor utilizing an estimated relative therapeutic exogenous substance concentration;

calculating a compensating dose of the therapeutic exogenous substance based on the estimated amount of the therapeutic exogenous substance to deliver adjusted by the insulin adjustment factor; and

causing the calculated compensating dose of the therapeutic exogenous substance to be expelled from a reservoir by a pump mechanism.

15. The method of embodiment 14, further comprising:
determining the estimated relative therapeutic exogenous substance concentration using a total daily amount of the therapeutic exogenous substance that maintained an analyte measurement value of a user within a range of a target analyte measurement setting.

16. The method of embodiment 14, wherein calculating the compensating dose of the therapeutic exogenous substance, further comprises:

obtaining, from a substance delivery history stored in a memory of the automatic drug delivery device, a total amount of the therapeutic exogenous substance that has been delivered to a user over a preset time period; and
determining, based on the obtained total amount of the therapeutic exogenous substance, a total daily amount of the therapeutic exogenous substance that maintains the user within range of a target analyte measurement setting.

17. The method of embodiment 16, further comprising:

obtaining, from the substance delivery history stored in the memory, a total amount of the therapeutic exogenous substance that has been delivered to the user over the preset time period;

calculating a per cycle delivery amount of using the total amount of the therapeutic exogenous substance that has delivered to the user over the preset time period;

obtaining a divisor by applying a tuning factor that corresponds to a predetermined number of delivery cycles to the calculated per cycle delivery amount;

dividing a sum of an amount past actual delivery of the therapeutic exogenous substance over a period of time corresponding to the tuning factor by the divisor; and

storing a quotient of the dividing as the estimated relative therapeutic exogenous substance concentration.

18. The method of embodiment 17, further comprising:

determining whether the estimated relative therapeutic exogenous substance concentration has a value within a maximum boundary and a minimum boundary; and

in response to the value of the estimated relative therapeutic exogenous substance concentration being within the maximum boundary and the minimum boundary, setting the insulin adjustment factor to be equal to the estimated relative therapeutic exogenous substance concentration.

19. The method of embodiment 17, further comprising:

determining whether the estimated relative therapeutic exogenous substance concentration has a value within a maximum boundary and a minimum boundary; and

in response to the value of the estimated relative therapeutic exogenous substance concentration being greater than the maximum boundary, setting the insulin adjustment factor to be equal to the maximum boundary.

20. The method of embodiment 17, further comprising:

determining whether the estimated relative therapeutic exogenous substance concentration has a value within a maximum boundary and a minimum boundary; and

in response to the value of the estimated relative therapeutic exogenous substance concentration being less than a value of the minimum boundary, setting the insulin adjustment factor to be equal to the value of the minimum boundary.

21. The method of embodiment 17, further comprising:

determining whether the estimated relative therapeutic exogenous substance concentration has a value within a maximum boundary and a minimum boundary; and

in response to the value of the estimated relative therapeutic exogenous substance concentration being less than the maximum boundary but greater than the minimum boundary, setting the insulin adjustment factor to be equal to the value of the estimated relative therapeutic exogenous substance concentration.

22. An automatic drug delivery device, comprising:

a processor,
a reservoir configured to contain a therapeutic exogenous substance;
a pump mechanism communicatively coupled to the processor, and that is fluidically coupled to the reservoir; and
a memory coupled to the processor and configured to store a substance delivery history and programming code that, when executed by the processor, configure the drug delivery device to:

calculate a substance correction constraint based on an analyte measurement value, a user's target analyte measurement setting, and a substance sensitivity factor;
determine a maximum substance concentration based on the substance correction constraint;
determine an estimated relative therapeutic exogenous substance concentration based on delivery of the therapeutic exogenous substance over a set number of delivery cycles;
determine an insulin adjustment factor using the maximum substance concentration and the estimated relative therapeutic exogenous substance concentration;
determine an amount of a therapeutic exogenous substance to deliver based on an analyte measurement value of a user;
calculate a compensating dose of the therapeutic exogenous substance based the amount of the therapeutic exogenous substance to deliver and the insulin adjustment factor; and
causing the calculated compensating dose of the therapeutic exogenous substance to be expelled from the reservoir by the pump mechanism.

23. The automatic drug delivery device of embodiment 22, wherein the drug delivery device is further configured to:
receive the analyte measurement value from an analyte sensor that is communicatively coupled to the processor.

24. The automatic drug delivery device of embodiment 22, wherein the drug delivery device is further configured to:
retrieve the analyte measurement value from a memory that is communicatively coupled to the processor, wherein the analyte measurement value retrieved from the memory is an estimate of the analyte measurement value based on previous analyte measurement values received from an analyte sensor.

25. The automatic drug delivery device of embodiment 22, wherein the drug delivery device, when calculating the substance correction constraint, is further configured to:

determine a difference between the analyte measurement value and a target analyte measurement setting of a user of the automatic drug delivery device; and
divide the difference by the substance sensitivity factor, wherein a quotient resulting from the dividing is the substance correction constraint.

26. The automatic drug delivery device of embodiment 22, wherein the drug delivery device, when determining the maximum substance concentration based on the substance correction constraint, is further configured to:

determine a maximum between the substance correction constraint and a substance on board parameter;
divide the substance correction constraint by the determined maximum;
determine a minimum between a constant term and a quotient of dividing the substance correction constraint by the determined maximum;
multiply determined minimum by a predetermined factor;
add a primary value to a result of the multiplying to obtain the maximum substance concentration.

27. The automatic drug delivery device of embodiment 22, wherein the drug delivery device when determining the insulin adjustment factor utilizing the substance correction constraint, is further configured to:

obtain the maximum substance concentration that was calculated using the substance correction constraint;
determine a minimum between estimated relative insulin concentration and the maximum substance concentration;
determine a maximum the minimum and a lower correction boundary; and

set the determined maximum as the insulin adjustment factor.

28. The automatic drug delivery device of embodiment 22, wherein the programming code further configures the drug delivery device to:
determine a maximum adjustment for the insulin adjustment factor, wherein the determining the maximum adjustment includes:

calculate a per cycle amount of the therapeutic exogenous substance delivered over a preset time;
multiply the per cycle amount, a tuning factor and a maximum threshold value together;
determine an amount of the therapeutic exogenous substance delivered over a period of time equal to the tuning factor;
compare a result from the multiplying to the determined amount of the therapeutic exogenous substance delivered over the period of time equal to the tuning factor; and
based on a result of the comparing, set the maximum adjustment of the insulin adjustment factor.

29. The automatic drug delivery device of embodiment 28, wherein the automatic drug delivery device when setting the maximum adjustment of the insulin adjustment factor is further configured to:
in response to the result of the comparing indicate that the result is greater than the determined amount of the therapeutic exogenous substance delivered over the period of time equal to the tuning factor, setting the insulin adjustment factor as determined to account for production of an endogenous insulin related to the therapeutic exogenous substance administered by the automatic drug delivery device.

30. The automatic drug delivery device of embodiment 28, wherein the automatic drug delivery device when setting the maximum adjustment of the insulin adjustment factor is further configured to:
in response to the result of the comparing indicate that the result is less than or equal to the determined amount of the therapeutic exogenous substance delivered over the period of time equal to the tuning factor, setting the insulin adjustment factor to one.

31. The automatic drug delivery device of embodiment 22, wherein the drug delivery device is further configured to:
in response to an analyte measurement value of the user being within a range of a target analyte measurement setting of the user, adjust the insulin adjustment factor calculated using the estimated relative therapeutic exogenous substance concentration and a preset maximum threshold.

32. The automatic drug delivery device of embodiment 22, wherein a total amount of the therapeutic exogenous substance may be limited based on a predetermined setting.

33. The automatic drug delivery device of embodiment 22, wherein the determination of the maximum substance concentration in addition to the substance correction constraint includes a minimum function and a preset percentage weighting factor for an output of the minimum function.

34. A method, comprising:

obtaining, by a processor of an automatic drug delivery device, an analyte measurement value of a user;

calculating a substance correction constraint based on the analyte measurement value, target analyte measurement setting of the user, and a substance sensitivity factor;

determining a maximum substance concentration based on the substance correction constraint;

determining an insulin adjustment factor to account for production of an endogenous insulin related to a therapeutic exogenous substance administered by the automatic drug delivery device;

calculating a compensating dose of the therapeutic exogenous substance based on an estimated amount of the therapeutic exogenous substance to deliver adjusted by the insulin adjustment factor; and

expelling the compensating dose of the therapeutic exogenous substance from a reservoir by a pump mechanism.

35. The method of embodiment 34, wherein obtaining the analyte measurement value, further comprises:
receiving the analyte measurement value from an analyte sensor that is communicatively coupled to the processor.

36. The method of embodiment 34, wherein obtaining the analyte measurement value, further comprises:
retrieving the analyte measurement value from a memory that is communicatively coupled to the processor, wherein the analyte measurement value retrieved from the memory is an estimate of the analyte measurement value based on previous analyte measurement values received from an analyte sensor.

37. The method of embodiment 34, wherein calculating the substance correction constraint further comprises:

determining a difference between the analyte measurement value and a target analyte measurement setting of the user; and

dividing the difference by the substance sensitivity factor, wherein a quotient resulting from the dividing is the substance correction constraint.

38. The method of embodiment 34, wherein determining the maximum substance concentration based on the substance correction constraint, further comprises:

determining a maximum between the substance correction constraint and a substance on board parameter;

dividing the substance correction constraint by the determined maximum;

determining a minimum between a constant term and a quotient of the dividing of the substance correction constraint by the determined maximum;

multiplying determined minimum by a predetermined factor;

adding a primary value to a result of the multiplying to obtain the maximum substance concentration.

39. The method of embodiment 34, wherein determining the insulin adjustment factor, further comprises:

obtaining the maximum substance concentration that was calculated using the substance correction constraint;

determining a minimum between estimated relative insulin concentration and the maximum substance concentration;

determining a maximum between the minimum and a lower correction boundary; and

setting the determined maximum as the insulin adjustment factor.

40. The method of embodiment 34, further comprising:
determining a maximum adjustment for the insulin adjustment factor, wherein the determining of the maximum adjustment for the insulin adjustment factor, includes:

calculating a per cycle amount of the therapeutic exogenous substance delivered over a preset time;
multiplying the per cycle amount, a tuning factor and a maximum threshold value together;
determining an amount of the therapeutic exogenous substance delivered over a period of time equal to the tuning factor;
comparing a result from the multiplying to the determined amount of the therapeutic exogenous substance delivered over the period of time equal to the tuning factor; and
based on a result of the comparing, setting the maximum adjustment of the insulin adjustment factor.

41. The method of embodiment 40, wherein setting the maximum adjustment of the insulin adjustment factor further comprises:
in response to result of the comparing indicating that the result is greater than the determined amount of the therapeutic exogenous substance delivered over the period of time equal to the tuning factor, setting the insulin adjustment factor as determined to account for the production of the endogenous insulin related to the therapeutic exogenous substance administered by the automatic drug delivery device.

42. The method of embodiment 40, wherein setting the maximum adjustment of the insulin adjustment factor further comprises:
in response to result of the comparing indicating that the result is less than or equal to the determined amount of the therapeutic exogenous substance delivered over a period of time equal to the tuning factor, setting the insulin adjustment factor to one.

43. A non-transitory computer-readable storage medium, the computer-readable storage medium including instructions that when executed by a processor, cause the processor to:

obtain, by a processor of an automatic drug delivery device, an analyte measurement value of a user;
calculate a substance correction constraint based on the analyte measurement value, a target analyte measurement setting of the user, and a substance sensitivity factor;
determine a maximum substance concentration based on the substance correction constraint;
determine an insulin adjustment factor utilizing the substance correction constraint;
calculate a compensating dose of the therapeutic exogenous substance based on an estimated amount of the therapeutic exogenous substance to deliver adjusted by the insulin adjustment factor; and
expel the compensating dose of the therapeutic exogenous substance from a reservoir by a pump mechanism of a drug delivery device.

44. The computer-readable storage medium of embodiment 43, further comprise instructions that when executed to obtain the analyte measurement value, cause the processor to:
receive the analyte measurement value from an analyte sensor that is communicatively coupled to the processor.

45. The computer-readable storage medium of embodiment 43, further comprises instructions that when executed to obtain the analyte measurement value, cause the processor to:
retrieve the analyte measurement value from a memory that is communicatively coupled to the processor, wherein the analyte measurement value retrieved from the memory is an estimate of the analyte measurement value based on previous analyte measurement values received from an analyte sensor.

46. The computer-readable storage medium of embodiment 43, further comprises instructions that when executed to calculate the substance correction constraint, cause the processor to:

determine a difference between the analyte measurement value and a target analyte measurement setting of a user of the automatic drug delivery device; and
divide the difference by the substance sensitivity factor, wherein a quotient resulting from the dividing is the substance correction constraint.

47. The computer-readable storage medium of embodiment 43, further comprises instructions that when executed to determine the maximum substance concentration based on the substance correction constraint, cause the processor to:

determine a maximum between the substance correction constraint and a substance on board parameter;
divide the substance correction constraint by the determined maximum;
determine a minimum between a constant term and a quotient of the dividing of the substance correction constraint by the determined maximum;
multiply determined minimum by a predetermined factor;
add a primary value to a result of the multiplying to obtain the maximum substance concentration.

48. The computer-readable storage medium of embodiment 43, further comprises instructions that when executed to determine the insulin adjustment factor utilize the substance correction constraint, cause the processor to:

obtain the maximum substance concentration that was calculated using the substance correction constraint;

determine a minimum between estimated relative insulin concentration and the maximum substance concentration;

determine a maximum of the determined minimum and a lower correction boundary; and

set the determined maximum as the insulin adjustment factor.

49. The computer-readable storage medium of embodiment 43, further comprises instructions that when executed, causes the processor to:

determine a maximum adjustment for the insulin adjustment factor, wherein the processor determines the maximum adjustment for the insulin adjustment factor by:

calculating a per cycle amount of the therapeutic exogenous substance delivered over a preset time;
multiplying the per cycle amount, a tuning factor and a maximum threshold value together;
determining an amount of the therapeutic exogenous substance delivered over a period of time equal to the tuning factor;
comparing a result from the multiplying to the determined amount of the therapeutic exogenous substance delivered over the period of time equal to the tuning factor; and
based on a result of the comparing, setting the maximum adjustment of the insulin adjustment factor.

50. The computer-readable storage medium of embodiment 49, further comprises instructions that when executed to set the maximum adjustment of the insulin adjustment factor cause the processor to:
in response to result of the comparing indicating that the result is greater than the determined amount of the therapeutic exogenous substance delivered over the period of time equal to the tuning factor, set the insulin adjustment factor as determined to account for production of endogenous insulin related to the therapeutic exogenous substance administered by the automatic drug delivery device.

51. The computer-readable storage medium of embodiment 49, further comprises instructions that when executed to set the maximum adjustment of the insulin adjustment factor cause the processor to:
in response to result of the comparing indicating that the result is less than or equal to the determined amount of the therapeutic exogenous substance delivered over a period of time equal to the tuning factor, set the insulin adjustment factor to one.

52. An automatic drug delivery system, comprising:

a processor,

a reservoir configured to contain insulin;

a pump mechanism communicatively coupled to the processor, and fluidically coupled to the reservoir; and

a memory coupled to the processor and configured to store an insulin delivery history and programming code that, when executed by the processor, configure the processor to:

estimate an amount of the insulin to deliver;

determine an insulin adjustment factor utilizing an estimated relative insulin concentration;

calculate a compensating dose of the insulin based on the estimated amount of the insulin to deliver that is adjusted by the insulin adjustment factor; and

causing the calculated compensating dose of the insulin to be expelled from the reservoir by the pump mechanism.

53. The automatic drug delivery system of embodiment 52, wherein the processor when calculating the compensating dose of the insulin is further configured to:

modify the estimated amount of the insulin using the insulin adjustment factor.

54. The automatic drug delivery system of embodiment 52, wherein the processor when calculating the compensating dose of the insulin is further configured to:

obtain, from the insulin delivery history stored in the memory, a total amount of the insulin that has been delivered to a user over a preset time period; and

determine, based on the obtained total amount of the insulin, a total daily amount of the insulin that maintains the user within range of a target analyte measurement setting.

55. The automatic drug delivery system of embodiment 52, wherein the processor, when determining the correction factor utilizing the estimated relative insulin concentration, is further configured to:

determine whether the estimated relative insulin concentration has a value within a maximum boundary and a minimum boundary; and

in response to the value of the estimated relative insulin concentration being within the maximum boundary and the minimum boundary, set the insulin adjustment factor to be equal to the estimated relative insulin concentration.

56. The automatic drug delivery system of embodiment 52, wherein the processor is further configured to:

determine the estimated relative insulin concentration using a total daily amount of the analyte that maintained an analyte measurement value of a user within a range of a target analyte measurement setting of the user.

57. The automatic drug delivery system of embodiment 52, wherein the processor is further configured to:

determine a sum of an amount past actual delivery of the insulin over a predetermined number of delivery cycles;

calculate a per cycle delivery amount of using a total amount of the insulin that has been delivered from the reservoir over a preset time period;

apply a tuning factor that corresponds to the predetermined number of delivery cycles to the calculated per cycle delivery amount to obtain a divisor;

divide the sum of an amount past actual delivery of the insulin over a predetermined number of delivery cycles by the obtained divisor; and

store a quotient of the dividing that is the estimated relative insulin concentration.

58. The automatic drug delivery system of embodiment 57, wherein the processor is further configured to:

determine a minimum value between the estimated relative insulin concentration or a maximum insulin adjustment factor;

based on the determined minimum, determine a maximum value between the estimated relative insulin concentration and a minimum boundary; and

in response to a value of the estimated relative insulin concentration being greater than the minimum boundary, set the insulin adjustment factor to be equal to a value of the estimated relative insulin concentration.

59. The automatic drug delivery system of embodiment 57, wherein the processor is further configured to:

determine whether the estimated relative insulin concentration has a value within a maximum boundary and a minimum boundary; and

in response to the value of the estimated relative insulin concentration being greater than the maximum boundary, set the insulin adjustment factor to be equal to a maximum insulin adjustment factor.

60. The automatic drug delivery system of embodiment 57, wherein the processor is further configured to:

determine whether the estimated relative insulin concentration has a value within a maximum boundary and a minimum boundary; and in response to the value of the estimated relative insulin concentration being less than the minimum boundary, set the insulin adjustment factor to be equal to a minimum insulin adjustment factor.

61. The automatic drug delivery system of embodiment 52, wherein the processor is further configured to:
determine a maximum adjustment of the insulin adjustment factor.

62. The automatic drug delivery system of embodiment 61, wherein the processor, when determining the maximum adjustment of the insulin adjustment factor is further configured to:

calculate a per cycle amount of the insulin delivered over a preset time;
multiply the per cycle amount, a tuning factor and a maximum threshold value together;
determine an amount of the insulin delivered over a period of time equal to the tuning factor;
compare a result from the multiplying to the determined amount of the insulin delivered over the period of time equal to the tuning factor; and
based on a result of the comparing, set the maximum adjustment of the insulin adjustment factor.

63. The automatic drug delivery system of embodiment 62, wherein setting the maximum adjustment of the insulin adjustment factor further comprises:
in response to result of the comparing indicating that the result is greater than the determined amount of the insulin delivered over the period of time equal to the tuning factor, set the insulin adjustment factor as determined by utilizing the estimated relative insulin concentration.

64. The automatic drug delivery system of embodiment 62, wherein setting the maximum adjustment of the insulin adjustment factor further comprises:
in response to result of the comparing indicating that the result is less than or equal to the determined amount of the insulin delivered over the period of time equal to the tuning factor, set the insulin adjustment factor to one.

Although the present invention is defined in the attached claims, it should be understood that the present invention can also (alternatively) be defined in accordance with the following embodiments:

1. An automatic drug delivery device, comprising:

a processor,
a reservoir configured to contain a therapeutic exogenous substance;
a pump mechanism communicatively coupled to the processor, and fluidically coupled to the reservoir; and
a memory coupled to the processor and configured to store a substance delivery history and programming code that, when executed by the processor, cause the drug delivery device to:

estimate an amount of the therapeutic exogenous substance to deliver to a user;
determine an insulin adjustment factor utilizing an estimated relative therapeutic exogenous substance concentration;
calculate a compensating dose of the therapeutic exogenous substance based on the estimated amount of the therapeutic exogenous substance to deliver that is adjusted by the insulin adjustment factor; and
causing the calculated compensating dose of the therapeutic exogenous substance to be expelled from the reservoir by the pump mechanism.

2. The automatic drug delivery device of embodiment 1, wherein the processor when calculating the compensating dose of the therapeutic exogenous substance is further configured to:
modify the estimated amount of the therapeutic exogenous substance using the insulin adjustment factor.

3. The automatic drug delivery device of embodiment 1, wherein the processor when calculating the compensating dose of the therapeutic exogenous substance is further configured to:

obtain, from the substance delivery history stored in the memory, a total amount of the therapeutic exogenous substance that has been delivered to the user over a preset time period; and

determine, based on the obtained total amount of the therapeutic exogenous substance, a total daily amount of the therapeutic exogenous substance that maintains the user within range of a target analyte measurement setting.

4. The automatic drug delivery device of embodiment 1, wherein the processor, when determining the insulin adjustment factor utilizing the estimated relative therapeutic exogenous substance concentration, is further configured to:

determine whether the estimated relative therapeutic exogenous substance concentration has a value within a maximum boundary and a minimum boundary; and
in response to the value of the estimated relative therapeutic exogenous substance concentration being within the maximum boundary and the minimum boundary, set the insulin adjustment factor to be equal to the therapeutic exogenous substance concentration.

5. The automatic drug delivery device of embodiment 1, wherein the processor is further configured to:
determine the estimated relative therapeutic exogenous substance concentration using a total daily amount of the therapeutic exogenous substance that maintained an analyte measurement value of the user within a range of a target analyte measurement setting.

6. The automatic drug delivery device of embodiment 1, wherein the processor is further configured to:

determine a sum of an amount past actual delivery of the therapeutic exogenous substance over a predetermined number of delivery cycles;

calculate a per cycle delivery amount of using a total amount of the therapeutic exogenous substance that has delivered to the user over a preset time period;

apply a tuning factor that corresponds to the predetermined number of delivery cycles to the calculated per cycle delivery amount to obtain a divisor;

divide the sum of an amount past actual delivery of the therapeutic exogenous substance over a predetermined number of delivery cycles by the obtained divisor; and

store a quotient of the dividing that is the estimated relative therapeutic exogenous substance concentration.

7. The automatic drug delivery device of embodiment 6, wherein the processor is further configured to:

determine a minimum value between the estimated relative therapeutic exogenous substance concentration or a maximum insulin adjustment factor;

based on the determined minimum, determine a maximum value between the estimated relative therapeutic exogenous substance concentration and a minimum boundary; and

in response to a value of the estimated relative therapeutic exogenous substance concentration being greater than the minimum boundary, set the insulin adjustment factor to be equal to the value of the estimated relative therapeutic exogenous substance concentration.

8. The automatic drug delivery device of embodiment 6, wherein the processor is further configured to:

determine whether the estimated relative therapeutic exogenous substance concentration has a value within a maximum boundary and a minimum boundary; and

in response to the value of the estimated relative therapeutic exogenous substance concentration being greater than the maximum boundary, set the insulin adjustment factor to be equal to a maximum insulin adjustment factor, or

determine whether the estimated relative therapeutic exogenous substance concentration has a value within a maximum boundary and a minimum boundary; and

in response to the value of the estimated relative therapeutic exogenous substance concentration being less than the minimum boundary, set the insulin adjustment factor to be equal to the minimum boundary.

9. The automatic drug delivery device of embodiment 1, wherein the processor is further configured to:
determine a maximum adjustment of the insulin adjustment factor, wherein when determining the maximum adjustment of the insulin adjustment factor, the processor is further configured to:

calculate a per cycle amount of the therapeutic exogenous substance delivered over a preset time;

multiply the per cycle amount, a tuning factor and a maximum threshold value together;

determine an amount of the therapeutic exogenous substance delivered over a period of time equal to the tuning factor;

compare a result from the multiplying to the determined amount of the therapeutic exogenous substance delivered over the period of time equal to the tuning factor; and

based on a result of the comparing, set the maximum adjustment of the insulin adjustment factor.

10. The automatic drug delivery device of embodiment 9, wherein setting the maximum adjustment of the insulin adjustment factor further comprises:

in response to result of the comparing indicating that the result is greater than the determined amount of the therapeutic exogenous substance delivered over the period of time equal to the tuning factor, set the insulin adjustment factor as determined to account for production of an endogenous insulin related to the therapeutic exogenous substance administered by the automatic drug delivery device, or

in response to result of the comparing indicating that the result is less than or equal to the determined amount of the therapeutic exogenous substance delivered over a period of time equal to the tuning factor, set the insulin adjustment factor to one.

11. A method, comprising:

estimating, by a processor of an automatic drug delivery device, an amount of a therapeutic exogenous substance to deliver;
determining an insulin adjustment factor utilizing an estimated relative therapeutic exogenous substance concentration;
calculating a compensating dose of the therapeutic exogenous substance based on the estimated amount of the therapeutic exogenous substance to deliver adjusted by the insulin adjustment factor; and
causing the calculated compensating dose of the therapeutic exogenous substance to be expelled from a reservoir by a pump mechanism.

12. The method of embodiment **11,** wherein calculating the compensating dose of the therapeutic exogenous substance, further comprises:

obtaining, from a substance delivery history stored in a memory of the automatic drug delivery device, a total amount of the therapeutic exogenous substance that has been delivered to a user over a preset time period; and
determining, based on the obtained total amount of the therapeutic exogenous substance, a total daily amount of the therapeutic exogenous substance that maintains the user within range of a target analyte measurement setting.

13. The method of embodiment 11, further comprising:
determining an estimated relative therapeutic exogenous substance concentration using a total daily amount of the therapeutic exogenous substance that maintained an analyte measurement value of a user within a range of a target analyte measurement setting.

14. The method of embodiment 13, further comprising:

determining whether the estimated relative therapeutic exogenous substance concentration has a value within a maximum boundary and a minimum boundary; and
in response to the value of the estimated relative therapeutic exogenous substance concentration being within the maximum boundary and the minimum boundary,
setting the insulin adjustment factor to be equal to the estimated relative therapeutic exogenous substance concentration, or
determining whether the estimated relative therapeutic exogenous substance concentration has a value within a maximum boundary and a minimum boundary; and
in response to the value of the estimated relative therapeutic exogenous substance concentration being greater than the maximum boundary, setting the insulin adjustment factor to be equal to the maximum boundary, or
determining whether the estimated relative therapeutic exogenous substance concentration has a value within a maximum boundary and a minimum boundary; and
in response to the value of the estimated relative therapeutic exogenous substance concentration being less than a value of the minimum boundary, setting the insulin adjustment factor to be equal to the value of the minimum boundary.

**Claims**

1. An automatic drug delivery device, comprising:

a processor,
a reservoir configured to contain a therapeutic exogenous substance;

a pump mechanism communicatively coupled to the processor, and fluidically coupled to the reservoir; and

a memory coupled to the processor and configured to store a substance delivery history and programming code that, when executed by the processor, cause the drug delivery device to:

estimate an amount of the therapeutic exogenous substance to deliver to a user;

calculate a compensating dose of the therapeutic exogenous substance to deliver based on an insulin adjustment factor, wherein the insulin adjustment factor accounts for an effect of the amount of therapeutic exogenous substance on the user's endogenous production of insulin; and

cause the calculated compensating dose of the therapeutic exogenous substance to be expelled from the reservoir by the pump mechanism.

2. The automatic drug delivery device of claim 1, wherein the drug delivery device is configured to bound the insulin adjustment factor to a value or a range of values based on an estimated endogenous insulin delivery value.

3. The automatic drug delivery device of claim 2, wherein the estimated endogenous insulin delivery value is determined based on an amount of endogenous insulin delivery that would occur without delivery of the amount of the therapeutic exogenous substance.

4. The automatic drug delivery device of claim 1, wherein the effect of the amount of therapeutic exogenous substance on the user's endogenous production of insulin is a decrease in endogenous insulin delivery.

5. The automatic drug delivery device of claim 1, wherein the drug delivery device is configured to determine a maximum insulin adjustment factor based on a total daily insulin (TDI) value of the user and calculate the compensating dose of the therapeutic exogenous substance while taking into account the maximum insulin adjustment factor.

6. The automatic drug delivery device of claim 1, wherein the drug delivery device is configured to limit the insulin adjustment factor to a value or range of values until a preset number of delivery cycles occur.

7. The automatic drug delivery device of claim 1, wherein the drug delivery device is configured to:

determine a constraint for the insulin adjustment factor based on a sum of past deliveries to the user and a threshold value; and

modify the insulin adjustment factor based on the constraint.

8. The automatic drug delivery device of claim 7, wherein the threshold value is a percentage of an amount of insulin delivery above an estimated total delivery based on a total daily insulin (TDI) value of the user.

9. The automatic drug delivery device of claim 1, wherein the drug delivery device is configured to calculate a per cycle amount of the therapeutic exogenous substance delivered over a period of time.

10. The automatic drug delivery device of claim 9, wherein the drug delivery device is configured to:

modify the per cycle amount with a tuning factor and maximum threshold value;

determine an amount of the therapeutic exogenous substance delivered over a period of time equal to the tuning factor;

compare the modified per cycle amount with the determined amount of the therapeutic exogenous substance;

based on the comparison, calculate a final insulin adjustment factor; and

calculate the compensating does based on the final insulin adjustment factor.

11. A computer-implemented method of calculating compensating doses of therapeutic exogenous substances using a processor of an automatic drug delivery device, comprising:

estimating an amount of a therapeutic exogenous substance to deliver to a user;

determining an insulin adjustment factor, wherein the insulin adjustment factor accounts for an effect of the amount of therapeutic exogenous substance on the user's endogenous production of insulin;

calculating a compensating dose of the therapeutic exogenous substance based on the estimated amount of the therapeutic exogenous substance to deliver adjusted by the insulin adjustment factor; and

causing the calculated compensating dose of the therapeutic exogenous substance to be delivered to the user.

12. The method of claim 11, further comprising bounding the insulin adjustment factor to a value or a range of values based on an estimated endogenous insulin delivery value.

**13.** The method of claim 12, wherein the estimated endogenous insulin delivery value is determined based on an amount of endogenous insulin delivery that would occur without delivery of the amount of the therapeutic exogenous substance.

**14.** The method of claim 11, wherein the effect of the amount of therapeutic exogenous substance on the user's endogenous production of insulin is a decrease in endogenous insulin delivery.

**15.** An automatic drug delivery system, comprising:

  a processor,
  a reservoir configured to contain a therapeutic exogenous substance;
  a pump mechanism communicatively coupled to the processor, and fluidically coupled to the reservoir; and
  a memory coupled to the processor and configured to store a substance delivery history and programming code that, when executed by the processor, cause the drug delivery device to:

    estimate an amount of the therapeutic exogenous substance to deliver to a user;
    calculate a compensating dose of the therapeutic exogenous substance to deliver based on an insulin adjustment factor, wherein the insulin adjustment factor accounts for an effect of the amount of therapeutic exogenous substance on the user's endogenous production of insulin; and
    cause the calculated compensating dose of the therapeutic exogenous substance to be expelled from the reservoir by the pump mechanism.

**FIG. 1**

FIG. 2

300

OBTAIN, BY A PROCESSOR OF AN AUTOMATIC DRUG DELIVERY DEVICE, AN ANALYTE MEASUREMENT VALUE OF A USER 302

↓

CALCULATE A SUBSTANCE CORRECTION CONSTRAINT BASED ON THE ANALYTE MEASUREMENT VALUE, A USER'S TARGET ANALYTE MEASUREMENT SETTING, AND A SUBSTANCE SENSITIVITY FACTOR 304

↓

DETERMINE A MAXIMUM SUBSTANCE CONCENTRATION BASED ON THE SUBSTANCE CORRECTION CONSTRAINT 306

↓

DETERMINE A CORRECTION FACTOR UTILIZING SUBSTANCE CORRECTION CONSTRAINT 308

↓

CALCULATE A COMPENSATING DOSE OF THE THERAPEUTIC EXOGENOUS SUBSTANCE BASED ON AN ESTIMATED AMOUNT OF THE THERAPEUTIC EXOGENOUS SUBSTANCE TO DELIVER OFFSET BY THE CORRECTION FACTOR 310

↓

EXPEL THE COMPENSATING DOSE OF THE THERAPEUTIC EXOGENOUS SUBSTANCE FROM THE RESERVOIR BY THE PUMP MECHANISM 312

**FIG. 3**

400

CALCULATE A PER CYCLE AMOUNT OF THE THERAPEUTIC
EXOGENOUS SUBSTANCE DELIVERED OVER A PRESET TIME
402

MULTIPLY THE PER CYCLE AMOUNT, A TUNING FACTOR AND
A MAXIMUM THRESHOLD VALUE TOGETHER 404

DETERMINE AN AMOUNT OF THE THERAPEUTIC EXOGENOUS
SUBSTANCE DELIVERED OVER A PERIOD OF TIME EQUAL TO
THE TUNING FACTOR 406

COMPARE THE RESULTANT FROM THE MULTIPLYING TO THE
DETERMINED AMOUNT OF THE THERAPEUTIC EXOGENOUS
SUBSTANCE DELIVERED OVER A PERIOD OF TIME EQUAL TO
THE TUNING FACTOR 408

BASED ON A RESULT OF THE COMPARING, SET THE
MAXIMUM ADJUSTMENT OF THE CORRECTION FACTOR 410

**FIG. 4**

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HOVORKA et al.** Glucose Turnover After Replacement of Usual Therapy by Insulin in Insulin-naive Type 2 Diabetes Subjects. *J Clin Endocrinol Metab*, 2014